# EUROPEAN PATENT APPLICATION

(11) **EP 0 985 388 A2**
(43) Date of publication of application: **15.03.2000**
(21) Application number: 99307170.3
(22) Date of filing: 10.09.1999
(51) Int. Cl.: A61F 2/50

(54) **Manufacturing methods for an artificial limb**

(30) Priority: 10.09.1998 GB 9819783
(71) Applicant: Watts, Robert John, Fordingbridge, Hampshire SP6 2PJ (GB)
(72) Inventor: Watts, Robert John, Fordingbridge, Hampshire SP6 2PJ (GB)
(74) Representative: Purvis, William Michael Cameron

(57) **Abstract**

A method of making a cover (34) for an artificial limb (1,20), the artificial limb being provided to replace at least a portion of a patient's missing limb, the method comprising the steps of: forming a skin coloured base layer for overlying the artificial limb, selecting one or more discrete outer layer portions (28) of a different tonal colour to that of the base layer; and attaching said outer layer portions to the base layer to provide one or more local colour variations in the colour of said cover so that the overall appearance of the cover is similar to that of the patient's skin.

## Description

This invention relates to manufacturing methods, and to articles manufactured thereby. One illustrative embodiment of the invention relates to a cover for an artificial limb, an orthosis and a method of manufacturing a cover for an artificial limb.

Artificial limbs are devices that are commonly used by amputees to replace missing body parts. They may be functional devices or purely cosmetic devices. Orthoses are devices which are usually employed to support and/or assist various parts of the human body that may be suffering some degree of disability or malformity.

The earliest artificial limbs were made of leather, wood and/or metal. Later artificial limbs have been made from aluminium alloys, and aluminium artificial limbs are still being manufactured today. Each of these previously proposed devices has its own set of associated advantages and disadvantages.

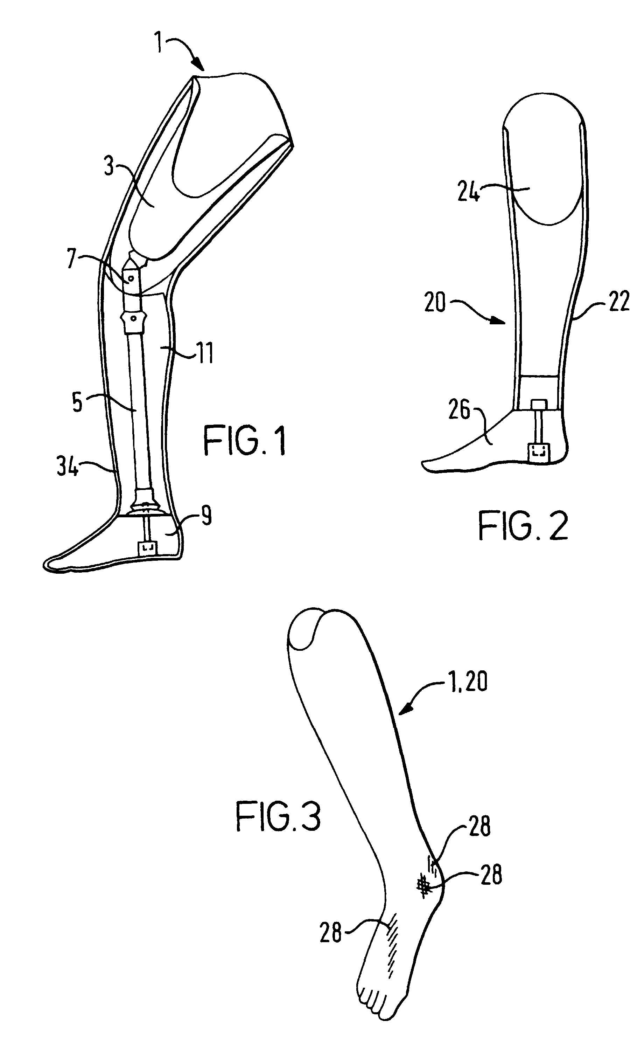

Some of these previously proposed devices include a leather socket which is intended to be in close proximity to the skin when in use. Leather is an advantageous material to employ as it has the ability to breath and therefore reduces the tendency of the residual limb (stump) to sweat. However, leather is expensive to work with and is not particularly resistant to wear. Other more modern devices are of closed cell plastics which whilst being better resistant to wear, do not allow the residual limb to breath. Aluminium limbs have recently been proposed as aluminium is very light, strong, and capable of dissipating heat (in contrast to plastics materials) thus keeping the residual limb cooler. However, aluminium limbs are difficult to shape into a life-like form, and are relatively expensive to produce. Another disadvantage associated with these previously proposed devices is that they are exoskeletal in design (see Figure 2).

In an effort to alleviate these problems, modern artificial limb designers have previously proposed adopting an endoskeletal format for prostheses. Endoskeletal artificial limbs typically comprise an internal skeleton and a cover provided thereover, gaps between the cover and skeleton being filled with a suitable supporting material such as expanded polystyrene foam.

The major manufacturers are also now recognising that endoskeletal limbs provide a standard method of attaching componentry that is appealing to the industry as a whole. As a result of this, components from different manufacturers can be assembled together giving a far wider choice of component characteristics for a particular prosthesis. Of particular benefit to the amputee is the fact that at the fitting stage (halfway through the manufacturing process) various knee joints and types of feet, for example, can be experimented with. Thus, if - for example - a knee joint does not suit the amputee's walking style then a different type can be easily attached. This was not possible with the previously proposed exoskeletal systems.

The endoskeletal system of artificial limb manufacture has also prompted the prosthetist (artificial limb fitter) to look more closely at the technical problem of how to make the limb appear more life-like. To date, however, no acceptable solutions to this problem have been proposed.

A further technical problem to be addressed is how to reduce creasing of the cover during use. Obviously, natural skin does not crease to an appreciable degree when a limb is flexed. Artificial limb covers, on the other hand, are prone to pronounced creasing that immediately indicates, even to the casual observer, that the limb in question is a prosthesis. To address this problem, it has been proposed to cover the artificial limb cover with a stocking, such as a nylon stocking, in order to reduce the amount of creasing. However, the use of a stocking is not an acceptable solution to this particular technical problem as stockings do not appear life-like, and thus once again draw attention to the fact that the limb is a prosthesis.

The majority of artificial limbs produced today are designed with their mechanical characteristics being the prime concern. However, and as mentioned above, there are a growing number of amputees that also require their artificial limbs to look more like the body part that the artificial limb is to replace. The manufacture of realistic looking covers for artificial limbs has been developing for some years. More recently silicone has been used for this purpose, although other alternative materials will be immediately apparent to those persons skilled in this and other related arts.

Some previously proposed manufacturing methods for silicone cosmetic covers have involved the use of metal moulds. In such a process, a metal mould is built up in layers over a model by electrolysis. The metal mould can then be used for the slush-moulding of a cosmetic cover. Slush moulding involves thinning the silicone down to a state where it can be poured into the mould, and then pouring the thinned silicone out of the mould to leave, within the mould, a thin layer of silicone. The process is then repeated until the required thickness of silicone is achieved. This is disadvantageous, however, as initial costs are high.

Colouring of the silicone can be attained in three ways. Firstly, the outside of the cover can be painted with a solution of thinners and pigment, but this is disadvantageous as the colours tend to wear off quite quickly. Secondly, a translucent cover can be produced, and the inside of the cover can then be painted with the required colours using long brushes. This is disadvantageous, however, as it is very time consuming and thus expensive. Thirdly, the colour of the cover can be mixed into the pouring solution. This is a quick and cost effective way of making coloured covers but they suffer from the problem that they are not very similar to natural skin as they are all one colour.

The process of producing and painting silicone cosmetic covers using moulds is time consuming, and due to the fumes it produces, is not healthy for the technician even with sophisticated extraction apparatus. Another disadvantage is that a mould such as this will not produce a cover that is shaped to fit a particular individual. If one wanted to cater for a number of amputees, then it would either be necessary to have a large number of different moulds, or it would be necessary to produce a new mould for each customer. If the stock of moulds were to be limited then the choice to the customer of hand or foot sizes would be correspondingly limited. Accordingly, neither of these solutions are particularly attractive.

The present invention aims to address at least some of the technical problems associated with previously proposed devices.

One aspect of the invention provides a method of making a cover for an artificial limb, the artificial limb being provided to replace at least a portion of a patient's missing limb, the method comprising the steps of: forming a skin coloured base layer for overlying the artificial limb, selecting one or more discrete outer layer portions of a different tonal colour to that of the base layer; and attaching said outer layer portions to the base layer to provide one or more local colour variations in the colour of said cover so that the overall appearance of the cover is similar to that of the patient's skin.

Another aspect of the invention provides a cover for an artificial limb, the cover comprising a skin-coloured base layer and one or more discrete outer layer sections attached thereto, the one or more outer layer sections having a different tonal colouring to that of the base layer to provide a local colour variation in the vicinity of each of said one or more discrete outer layer sections, said local colour variation combining with the colour of the base layer to provide an overall appearance for the cover that is similar to that of human skin

A further aspect of the invention provides a manufacturing method for an artificial limb that is provided to replace at least part of a patient's missing limb, the method comprising the steps of: (a) providing an artificial limb having a socket for fitting to the patient or to a remaining portion of the patient's missing limb; (b) adjusting the socket of the artificial limb to fit the patient or the remaining portion of the patient's missing limb; (c) reducing at a plurality of locations the peripheral circumference of the artificial limb until the circumference of said artificial limb at each of said plurality of locations is smaller than or equal to that of a corresponding human limb; and (d) fitting a cover as described herein to the artificial limb.

Embodiments of the invention will now be described, by way of example only, with reference to the accompanying figures, in which:
Figure 1 is a schematic cross-sectional view of a transfemoral endoskeletal prosthesis;
Figure 2 is a schematic cross-sectional view of a transtibial exoskeletal prosthesis;
Figure 3 is a schematic perspective view of the outside of a transtibial prosthesis;
Figure 4 is a schematic perspective view of the outside of another transtibial prosthesis; and
Figure 5 is a schematic cross-sectional view of a cover for an endo- or exo-skeletal prosthesis.
Figure 6 is a schematic representation of an ankle-foot orthosis.

The following description relates to an illustrative example of the manufacture of an endoskeletal prosthesis that has a silicone cover provided thereover. It should be understood that the present application is not limited to endo- or exo- skeletal prostheses, and that is not limited to *silicone* cosmetic covers as a large variety of different materials may be used. The teachings of the present invention may also be applied to orthoses, for example. It should furthermore be noted that the teachings of the invention may be applied to prostheses that serve to replace a portion of a patient's limb (i.e. prostheses for attachment to a residual stump) or to prostheses that serve to replace a patient's limb in its entirety (i.e. prostheses for attachment to the patient's torso).

As mentioned above, Figure 1 is a schematic cross-sectional view of a transfemoral endoskeletal prosthesis 1. As shown , the prosthesis comprises a socket 3 into which a user's stump may be fitted, a shin tube 5 fitted to the socket by way of an adapter 7 and a foot 9 which is in turn fitted to the tube 5. The area surrounding the shin tube 5 is of foam 11 (as is later described in detail).

Figure 2 is a schematic cross-sectional view of a transtibial exoskeletal prosthesis 20. In this example, the prosthesis 20 comprises a rigid outer shell 22 to which a socket 24 and a foot 26 are attached. Figure 3 is a schematic perspective view of the outside of a transtibial prosthesis (1 or 20), which has had a number of differently coloured patches 28 applied to a cover fitted thereto in order to more closely simulate human skin. The outer shell 22 is of a material (for example wood or plastics or any other suitable material) that may be reduced in circumference to allow space for the cover to be fitted thereto.

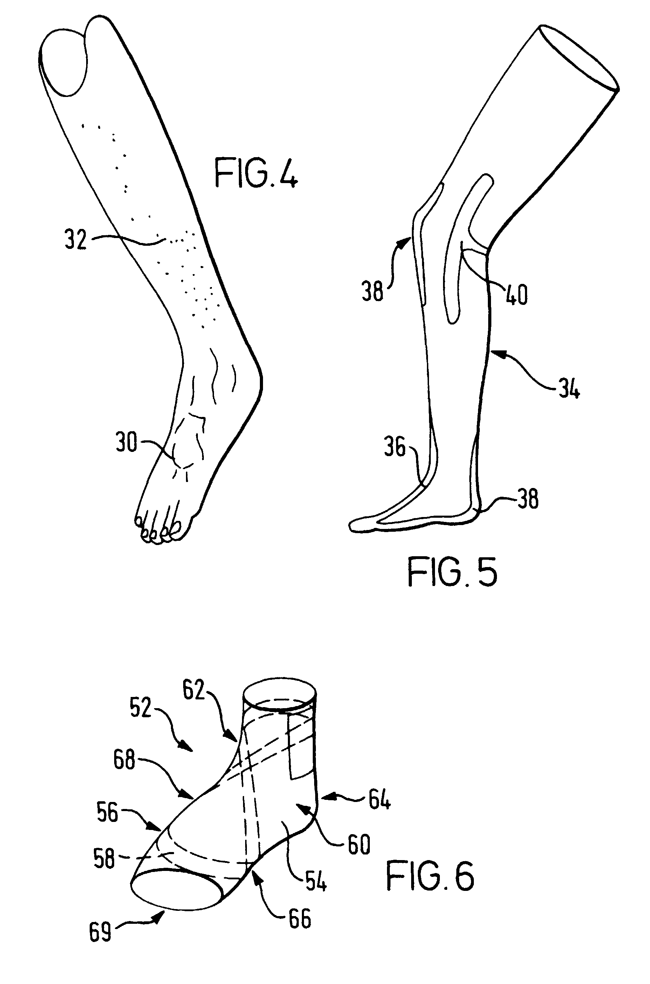

Figure 4 is a schematic perspective view of the outside of another transtibial prosthesis showing a variety of veins 30 and hair follicles 32 that have been formed therein; and Figure 5 is a schematic cross-sectional view of a cover 34 for an endo- or exo-skeletal prosthesis. Various regions 36 of the cover 34 have been thickened in order to reduce the amount of creasing of the cover during use.

Some suitable materials from which the cover 34, in this example, may be manufactured are:
1. Chlorosil parts A & B, an elastomer rubber crepe, as supplied by Otto Bock of Benelux B.V.;
2. Med-4055® two-part silicone elastomer as produced by NuSil Technology of the United States of America. This elastomer comprises dimethyl and methyvinyl siloxane copolymers and reinforcing silica; and
3. Dow Corning Silastic® Q7-4720 Biomedical Grade Elastomer ETR. This elastomer is a low durimeter, two-part, enhanced-tear-resistant (ETR) silicone elastomer that consists of dimethyl silcoxane copolymers and reinforcing silica. Silastic® Q7-4720 Biomedical Grade ETR is strained through a 200 mesh ( 75 micron) screen to ensure freedom from particle contamination. The elastomer is supplied as a two component kit ( part A & part B ), equal portions of which must be thoroughly blended together prior to use. The elastomer is then thermally cured via addition cure ( platinum cure) chemistry.

Other suitable materials will be apparent to those persons skilled in this and other arts.

The process starts by producing an artificial limb in either endoskeletal 1 or exoskeletal 20 form (see Figures 1 & 2 respectively). The process of producing a silicone cosmetic cover 34 can be applied to transtibial (below knee) and transhumeral (above knee) prostheses, transradial (below elbow) and transhumeral (above elbow) prostheses, regardless of whether they are in endo- or exo-skeletal form.

For the purposes of the following description an illustrative method for the manufacture of an endoskeletal transtibial prosthesis 1 will be detailed. To make the prosthesis 1 , a so-called "negative" cast is first made of the patient's residual limb using a plaster of paris bandage. If the patient should not have a residual limb, then a cast is taken of that part of the patient's torso to which the prosthesis is to be fitted.

Next, a liquid mixture of approximately 50 % plaster of paris powder and 50 % water is poured into the negative cast to form a so-called "positive" cast. The positive cast is then covered with a layer of plastics material. The layer is preferably in the region of 6mm thick and preferably of P.E.Lite^{TM}. or silicone or of both P.E.Lite^{TM} and silicone.

Six to ten layers of stockinette, fibreglass and carbon fibre tubing are then placed over the P.E.Lite^{TM} to form a socket 3 for the limb skeleton. A liquid acrylic resin is then vacuum formed into the layers of the socket, and the resin quickly solidifies, usually within the hour, to form a "first lamination" that forms the basis for the socket which is to be attached to the residual limb. The socket 3 is glued onto an adapter 7 which is connected to a shin tube 5 that is, in turn, connected to a foot 9 (see figure 1). As an alternative, the socket may be formed from polypropylene or from any other material known to persons skilled in the art.

The prosthesis 1 is now ready for fitting and can be fitted to, aligned and walked on by the amputee. When the prosthetist and amputee are satisfied with the socket fit and alignment of the prosthesis it is then sent for finishing, and an optional second lamination over the socket may be carried out if required for strength. The prosthesis skeleton is then covered with a foam, such as plasterzote^{TM} for example.

A profile of the amputee's sound side leg, in this case, is then taken giving shape, circumferences and widths in the front and side views. The technician or prosthetist uses this information, as well as offering up the prosthesis to the sound leg, to produce an artificial leg that is as near identical in shape and size to the sound leg as the alignment of the socket will allow. If the patient should be a double amputee (i.e. someone who has lost both legs or both arms), then the artificial leg may be sized and shaped by comparing it to the leg of a similarly sized person, for example a person of similar size and/or weight. References herein to the amputee's sound limb should therefore be construed to include the sound limb of another individual if the amputee has lost both arms or both legs.

The silicone cover can be made by one of two methods. In the first method, a duplicate "negative" cast of the prosthesis is taken and used to form a positive cast that is subsequently shaped and reduced to compensate for the additional thickness of a silicone cover. This method is preferably always used with transfemoral silicone covers.

In the second method (particularly preferable for transtibial orthoses), the outer surface of the prosthesis is reduced to provide a profile that compensates for the additional thickness of the silicone cover. The second, more preferable, method will now be described by way of example only.

To obtain the above mentioned profile using the second method, measurements are taken every 50 to 100mm downwards from the patella tendon of the amputee's sound limb and the artificial limb. Reference marks are then applied to the outside of the prosthesis identifying the distances at and below the patella tendon. The prosthesis is shaved down to the specified measurements, whilst retaining the shape of the sound side limb. Special attention is paid to the area at the back of the heel, especially the malleoli and post malleoli fossa's to ensure they are as identical to the sound side as possible. If a Flex Foot^{TM} (type of prosthetic foot) is to be used then approximately 10mm is shaved off the plantar surface of the prosthesis beneath the heel to compensate for heel deflexion of the silicone cosmetic cover on heel strike.

To prepare the prosthesis 1 for the silicone cosmetic cover 34, the circumferences of the prosthesis are now reduced by approximately 10mm or more or less to allow for the fitting of the cover. At the same time, shaping of the artificial foot 9 may be undertaken to give the artificial limb 1 approximately the same heel and foot width and depth as the sound side. The foot of the limb is approximately 2mm smaller all over to allow for the thickness of the silicone cover that is to be fitted thereover. The cover may be of a 20, 35 or 65 shore silicone type two-part biomedical elastomer with enhanced tear resistance, or any other suitable material.

With the amputee present, a suitable colour is selected by either using a colour swatch or by mixing a number of coloured pieces of silicone, or by providing a suitable quantity of silicone and applying pigments to the silicone until the required shade of colour is achieved. These colours are then built up and patched together. Colours typically vary under different lighting conditions and thus a room is used with north light strip lighting or other suitable lighting - such as a high powered video light - to reduce these effects. The selected coloured sample or swatches are then placed on a production chart.

The base silicone is then milled to the required colour and thickness and laid onto the prosthesis 1. If other colours are required then suitable pigments are applied to further pieces of silicone and these pieces are laid adjacent to the original piece of silicone and the two colours are blended together to achieve a gradual transfer from one colour to the next. The resulting silicone cosmetic cover 34 could therefore be made of several different single colours blended at their unions, see figure 3, or of a single generally skin-matched colour.

As mentioned above, the silicone cosmetic cover 34 can be made on a cast, in accordance with the first method, or directly on the prosthesis 1, in accordance with the second method as described above.

The artificial limb cover is then treated so that it more closely resembles a natural limb. As a first step, small strands 30 of, for example, green or blue (or of a green/blue mixture) silicone (typically in the order of 1mm thick) are buried beneath the surface of the silicone cover so that the strands are faintly visible through the cover. The small strands 30 of silicone are placed and glued onto the areas of prosthesis 1 or cast where they are required. The sheet of pigmented silicone is then placed over the prosthesis or cast and the various colours rolled and blended into each other. The silicone is then rolled thinner in the neighbourhood of the strands until the strands are faintly visible therethrough. The portion of the cover 34 in the area of the strand may then be slightly elevated to so that it looks more like a natural vein, see Figure 8.

If human skin is studied it can be seen that the overall colour of the skin is made up of a number differently coloured layers. To simulate this in a cover 34, a method known as "patching" is employed. In "patching", small pieces of silicone 28 ( which may be as small as 3mm in diameter and 0.5 mm thick) of different coloured silicones or silicones of different tonal colours are patched onto each other. Their edges are blended together, for example by using a small roller, to give a mottled multi-coloured layered effect that is similar to real human skin. Rolling the silicone in the manner described serves to thin the silicone and thus to increase its translucency and reduce the intensity, in the neighbourhood of the rolling, of any applied colour The small sections of silicone are preferably selected to be of a colour similar to that of colour variations in a patient's skin, or similar to colour variations of a corresponding sound limb of the patient. This method differs from previous procedures in that the patching is placed onto the prosthesis in the opposite way that silicone is applied to a mould (in other words, the small pieces of silicone are applied to the outer surface of the cover, whereas in a mould different colours are painted (or otherwise provided) on the inside surface of the cover). It also has the advantage over the moulding method in that after the silicone has been applied it can be observed and changed if it is not quite the required colour. This cannot be done in a mould.

The resulting cover will therefore comprise a base layer and one or more outer layer portions that have been attached thereto, and preferably blended therewith so as to provide a smooth transition from one colour to another.

For yet greater realism, the cover 34 may be provided with simulated hair follicles 32 to give the appearance that the limb has been shaved. These hair follicles resemble small dark pin pricks and are formed by pushing a small sharp implement, for example a sharpened matchstick, a depth of approximately 1mm into the silicone at several different places. A small amount of pigment is applied to the sharp end of the implement before it is pushed into the silicone, and this pigment is retained in the cover to give the appearance of a recently shaved hair follicle. If a hairy limb is required artificial or real hair is immersed in a primer solution to remove any greases or contaminants. The hair is then placed onto the surface of the prosthesis and rolled into the silicone. A vacuum bag ideally made of PVA is placed over the silicone and a vacuum applied to the prosthesis of approximately 0.7bar for a period of approximately one hour.

To reduce creasing of the silicone on the dorsal surface of the silicone and medial foot cover 34 at the walking stage of toe off, and in the achilles tendon region when the heel is placed on the ground, reinforcement of the silicone cover is carried out by using either a thicker piece of 35 shore silicone in the said areas or by incorporating a 65 shore stiffener. This thickening of the silicone can also be applied to stop creasing of the fingers of a transradial prosthesis. To stop creasing at the knee of a transfemeral silicone cosmetic cover; the knee area 38 at the front of the cover is thickened with 35 shore silicone or reinforced with a harder 65 shore. For example, in addition to the 1.5mm to 2.5mm thickness of the cover, the thickness of the knee area and especially the patella (knee cap) region can be increased by up to a depth of 10mm. Posterioraly, at the back of the knee, the silicone may be reduced in thickness to 1.5 mm. To ensure that creasing is minimal at either side of the knee when the knee is in the flexed position the cast or prosthesis over which the cover is made is preflexed at an angle of between 20° and 45° depending on the length of artificial limb and type of knee joint used. To restrict ballooning of the silicone either side of the knee, a rib 40 up to 1cm wide of 35 or 65 shore is applied in the lateral and medial aspects and is incorporated beneath the surface and joined together across the back with a similar strip of reinforcing silicone (see Fig 5). In addition to the preflexion the cast's or prosthesis' measurements are reduced. Measurements are taken of the amputee's contralateral side and the artificial limb every 100mm above and below the knee centre. A 10% reduction is made of these measurements, with in some smaller circumferences up to 16%. Reference marks are then applied to the cast or prosthesis identifying the distances above and below the knee centre. The cast or prosthesis is then shaved down to the modified measurements specified, yet retaining the shape of the sound side. Special attention is paid to the patella and knee area so that the two knees are as identical as possible.

It will be understood, of course, that embodiments of the invention have been described herein by way of example only and that modifications may be made within the scope of the invention.

For example, the teachings of the present invention may equally be applied to orthoses, and more particularly to silicone orthoses. Various illustrative orthoses are described in co-pending United Kingdom Patent Application Nos. 2330309 and 2330311 - the contents of which are incorporated herein by reference, and these devices may be rendered more life-like by applying the teachings of the present invention thereto.

For example, United Kingdom Patent Application No. 2330309 discloses a sock-like orthosis that may be made from silicone. The outside of the orthosis could be adapted using the principles of the present invention so that it more closely resembles human skin by adding veins, and/or areas of local colour difference, and/or hair follicles.

Figure 6 shows an orthosis such as that described in UK Patent Application No. 2330309. As shown, the orthosis 52 comprises a resiliently flexible sock-like structure 54 and a reinforcing means 56 (shown in ghost), which could be a tape 58 - the ends of which are joined together to form a figure-of-eight which passes under the instep, behind the ankle and crosses on the dorsal aspect of the foot. The reinforcing means may be formed integrally with the sock-like structure or, alternatively, the reinforcing means may be formed separately and subsequently inserted within the sock-like structure. The reinforcing means may alternatively comprise a rib running down at least a portion of the front of the patients foot.

In any event, it should be noted that the reinforcing means 56 can be dispensed with, i.e. need not be provided, if sufficient resistance to plantarflexion is provided by the sock-like structure 54. In any event, it is preferred for the resilience of the reinforcing means 56, if provided, to be greater than that of the sock-like structure 54.

In the arrangement shown, the orthosis 52 envelops a portion of the patients lower leg which preferably includes the medial malleolus 60 (the inside of the ankle) and the lateral malleolus 62 (the outside of the ankle), the calcaneum 64 (the heel), a portion of the plantar aspect 66 of the foot (the sole of the foot) and a portion of the dorsal aspect 68 of the foot (the back of the foot). In this embodiment, the toes 69 of the foot are not enveloped by the orthosis, although they could be enveloped if desired. It is preferred that the orthosis extends beyond the medial and lateral malleoli. The orthosis does not have to envelop the calcaneum 64 of the foot. In fact, it has been found that the orthosis 52 can be made significantly more comfortable if both the calcaneum 64 and toes 69 are not enveloped by the sock-like structure 64.

Broadly speaking, Figure 6 shows an ankle-foot orthosis for resisting planterflexion of a patient's foot, the orthosis comprising: a resiliently flexible sock-like structure enveloping in use at least a portion of a patients lower leg in the vicinity of the ankle and at least a portion of the plantar and dorsal aspects of the patients foot. The orthosis may be of silicone (or other similar material) and the outside of the orthosis could be adapted using the principles of the present invention so that it more closely resembles human skin by adding veins, and/or areas of local colour difference, and/or hair follicles, for example.

Broadly speaking, it can be seen from the above that aspects of the invention provide a cover for an artificial limb that comprises a skin-coloured base layer for overlying the limb, and one or more outer layer portions attached to the base layer. The outer layer portions have a different colour to that of the base layer, or a different tonal colour to that of the base layer. A further aspect of the invention relates to an orthosis having these features.

## Claims

1. A method of making a cover for an artificial limb, the artificial limb being provided to replace at least a portion of a patient's missing limb, the method comprising the steps of: forming a skin coloured base layer for overlying the artificial limb, selecting one or more discrete outer layer portions of a different tonal colour to that of the base layer; and attaching said outer layer portions to the base layer to provide one or more local colour variations in the colour of said cover so that the overall appearance of the cover is similar to that of the patient's skin.

2. A method according to Claim 1, wherein the one or more discrete outer layer sections are selected to have a colour similar to that of variations in colour of the skin of a corresponding sound limb of the patient, the one or more outer layer sections being attached to the cover to provide the artificial limb with an overall appearance that is similar to that of the patient's corresponding sound limb.

3. A method according to Claim 1 or 2, comprising the step of:
blending the periphery of said one or more outer layer portions with said base layer to provide a substantially smooth transition from one colour to another.

4. A method according to any preceding claim, comprising the steps of:
(i) providing one or more strands within said base layer or between said base layer and said one or more outer layer portions, each of said strands having a different colour than said base layer or said outer layer portions; and
(ii) blending said strands with said base layer or said outer layer portions to cause said strands to be visible therethrough.

5. A method according to Claim 4, comprising the step of elevating the surface of the cover with respect to the remainder of the cover in the neighbourhood of said one or more strands.

6. A method according to Claim 4 or 5, wherein said one or more strands are coloured blue, green or a mixture of blue and green.

7. A method according to any of preceding claim, comprising the step of forming a plurality of coloured spots on said cover, the spots resembling shaved hair follicles on human skin.

8. A method according to Claim 7, wherein said spots are formed by implanting dye or pigment at a plurality of locations on said cover.

9. A method according to Claim 8, wherein said cover is implanted with dye or pigment by pushing a dye or pigment covered pointed implement a predetermined depth into said cover.

10. A method according to any preceding claim, comprising the step of reinforcing one or more portions of said cover, said reinforced portions being less susceptible to creasing upon flexure than remaining portions of said cover.

11. A method according to Claim 10, wherein said reinforcing step comprises varying the thickness of the cover at those regions to be reinforced.

12. A method according to Claim 10, wherein said reinforcing step comprises replacing said portions of said cover to be reinforced with replacement cover portions of stiffer material.

13. A method according to any preceding claim, wherein said base layer and said one or more outer layer portions are of silicone.

14. A method according to any preceding claim, wherein said base layer and/or said one or more outer layer portions are of 20 and/or of 35 and/or of 65 shore silicone.

15. A manufacturing method for an artificial limb that is provided to replace at least part of a patient's missing limb, the method comprising the steps of:
(a) providing an artificial limb having a socket for fitting to the patient or to a remaining portion of the patient's missing limb;
(b) adjusting the socket of the artificial limb to fit the patient or the remaining portion of the patient's missing limb;
(c) reducing at a plurality of locations the peripheral circumference of the artificial limb until the circumference of said artificial limb at each of said plurality of locations is smaller than or equal to that of a corresponding human limb; and
(d) fitting a cover made in accordance with the method of any of Claims 1 to 14 to the artificial limb.

16. A method according to Claim 15, wherein said artificial limb has an exoskeleton onto which said cover is fittable.

17. A method according to Claim 15, wherein said artificial limb comprises an endoskeleton and supporting material formed thereon, said cover being fittable onto said supporting material.

18. A method according to any of Claims 15 to 17, wherein said corresponding human limb is a remaining limb of the patient.

19. A method according to any of Claims 15 to 18, wherein said flexible cover is formed on said artificial limb.

20. A method according to any of Claims 15 to 19, wherein said flexible cover is formed on a cast of said artificial limb.

21. A method according to Claim 20, wherein said cast is formed by taking a negative impression of said limb, and filling said negative impression with a filler to form a positive impression of said limb.

22. A method according to any of Claims 15 to 21, wherein said reducing step comprises reducing the peripheral circumference of said artificial limb by approximately 10mm.

23. A cover for an artificial limb, the cover comprising a skin-coloured base layer and one or more discrete outer layer sections attached thereto, the one or more outer layer sections having a different tonal colouring to that of the base layer to provide a local colour variation in the vicinity of each of said one or more discrete outer layer sections, said local colour variation combining with the colour of the base layer to provide an overall appearance for the cover that is similar to that of human skin.

24. A cover according to Claim 23, comprising one or more strands provided within said base layer or between said base layer and said one or more outer layer portions, wherein each of said strands has a different colour than said base layer or outer layer portions and is visible through the base layer or outer layer portions.

25. A cover according to Claim 24, wherein said one or more strands are coloured blue, green or a mixture of blue and green.

26. A cover or an orthosis according to Claim 24, wherein said one or more outer layer portions and/or said base layer are provided with a plurality of coloured spots, the spots resembling shaved hair follicles on human skin.

27. A method of making an orthosis for resisting planterflexion of a patient's foot, the method comprising the steps of: forming a skin coloured sock-like base layer for overlying the patient's foot, selecting one or more discrete outer layer portions of a different tonal colour to that of the base layer; and attaching said outer layer portions to the base layer to provide one or more local colour variations in the colour of said orthosis so that the overall appearance of the orthosis is similar to that of the patient's skin.
